# EUROPEAN PATENT APPLICATION

(11) **EP 4 310 712 A1**
(43) Date of publication of application: **24.01.2024**
(21) Application number: 22771519.0
(22) Date of filing: 17.03.2022
(51) Int. Cl.: G06F 21/60, G06F 21/62, G16H 10/60

(54) **PROVIDING DEVICE, PROVIDING METHOD, AND PROVIDING PROGRAM**

(30) Priority: 18.03.2021 JP 2021044694
(71) Applicant: NTT Communications Corporation, Tokyo 100-8019 (JP)
(72) Inventor: SAKURAI, Yoichi, Tokyo 100-8019 (JP); IZUMI, Hiroaki, Tokyo 100-8019 (JP); UMEDA, Takeru, Tokyo 100-8019 (JP); KOKUBO, Ryuta, Tokyo 100-8019 (JP); OTA, Mitsunori, Tokyo 100-8019 (JP)
(74) Representative: Hoffmann Eitle
(86) International application number: PCT/JP2022/012393
(87) International publication number: WO 2022/196774

(57) **Abstract**

A storage unit (14) stores patient information (14a) on each patient and examination-treatment information (14b) on examination and treatment on each patient dispersedly. An acquisition unit (15a) acquires consent information indicating consent for provision of the patient information (14a) and the examination-treatment information (14b) to a third-party organization from each patient and adds the consent information to the patient information (14a) in the storage unit (14). A generation unit (15b) generates provision information (14c) obtained by combining the patient information (14a) containing the consent information and the examination-treatment information (14b) with respect to each patient. A provision unit (15c) provides the generated provision information (14c) to the third-party organization in response to a request from the third-party organization.

## Description

### [Technical Field]

The present invention relates to a provision device, a provision method and a provision program.

### [Background Art]

In recent years, in the field of medicine, an electric patient-reported outcome (ePRO) technique by which a patient himself/herself registers daily information, such as pains and quality of sleep, as electric data has been expected. In this technique, a patient answers a questionnaire in a given form, thereby registering electric data in a system. The electric data that is collected in this manner is tallied and analyzed by a third-party organization, such as a medical institute or a pharmaceutical company, with consent of the patient and is utilized for development of new drugs and advanced medicine.

### [Citation List]

### [Non Patent Literature]

"ePRO(patient report outcome system)", [online], January 6th, 2021, Accelight Corporation, [searched on January 6th, 2021], Internet <URL: https://accelight.co.jp/works/product/product-epro/>

### [Summary of Invention]

### [Technical Problem]

The related technique sometimes has a difficulty in safely providing the collected electric data for analysis. For example, for the electric data of answers on which consent of the patient was obtained before the collection, consent for provision is newly obtained. A method of stopping provision of the electric data when consent cannot be obtained because the purpose of use in the third-party organization changes or the intention of the patient changes has not been established specifically.

The present invention was made in view of the above-described circumstances and an object of the invention is to safely provide electric data containing collected personal information for analysis.

### [Solution to Problem]

In order to solve the above-described problems and achieve the object, a provision device according to the present invention includes: a storage unit configured to store patient information on each patient and examination-treatment information on examination and treatment on each patient dispersedly; an acquisition unit configured to acquire consent information indicating consent for provision of the patient information and the examination-treatment information from each patient to a third-party organization and add the consent information to the patient information in the storage unit; a generation unit configured to generate provision information obtained by combining the patient information containing the consent information and the examination-treatment information with respect to each patient; and a provision unit configured to provide the generated provision information to the third-party organization in response to a request from the third-party organization.

### [Advantageous Effects of Invention]

According to the present invention, it is possible to safely provide electric data containing collected individual information for analysis.

### [Brief Description of Drawings]

FIG. 1 is a diagram for describing an overview of a provision device.
FIG. 2 is a schematic view exemplifying a schematic configuration of the provision device.
FIG. 3 is a diagram for describing a process by an acquisition unit.
FIG. 4 is a diagram for describing the process by the acquisition unit.
FIG. 5 is a diagram for describing a process by a generation unit.
FIG. 6 is a diagram for describing the process by the generation unit.
FIG. 7 is a flowchart illustrating a procedure of a provision process.
FIG. 8 is a flowchart illustrating the procedure of the provision process.
FIG. 9 is a diagram exemplifying a computer that executes a provision program.

### [Embodiments for Carrying Out the Invention]

With reference to the drawings, an embodiment of the present invention will be described in detail below. The embodiment does not limit the invention. In the illustration of the drawings, the same components are denoted with the same reference numerals and are presented.

### Overview of Provision Apparatus

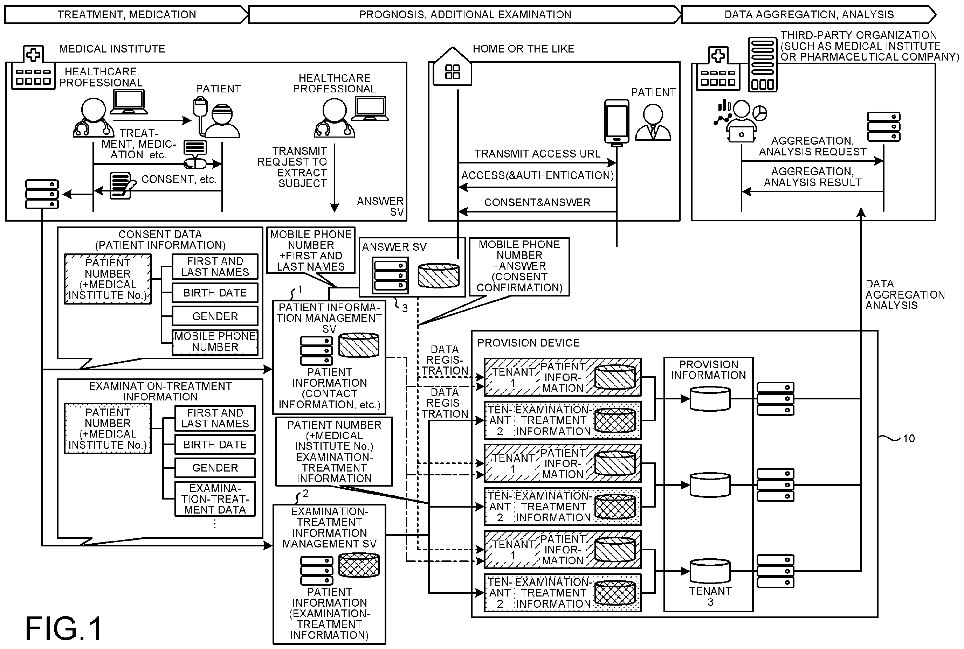

FIG. 1 is a diagram for describing an overview of a provision device. As exemplified in FIG. 1, a system including a provision device 10 is configured by further including a patient information management server (SV) 1, examination-treatment information management SV 2, and an answer SV 3.

The patient information management SV 1 manages, in addition to a patient number, first and last names, a birth date and gender that identify a patient, patient information containing contact information. For example, in a medical institute, initial data of consent data containing a patient number, first and last names, a birth date, gender, and a contact mobile phone number is generated with respect to a patient who receives explanation from a healthcare professional and gives consent for use of examination-treatment information, or the like, for analysis is generated and the initial data is registered as patient information in the patient information management SV 1.

The examination-treatment information management SV 2 manages, in addition to a patient number, first and last names, a birth date and gender, examination-treatment information containing examination-treatment data, such as content of treatment and a type and a method of medication. For example, in a medical institute, examination-treatment information containing a name, birth date, gender, and examination-treatment data is registered in the examination-treatment information management SV 2 by a healthcare professional in association with a patient number.

The answer SV 3 manages answers of a patient to a questionnaire and consent information for newly confirming consent for provision of examination-treatment information, or the like, to a third-party organization. For example, answers of a patient to a questionnaire for a follow-up at home and consent information indicating consent for provision of the answers to the questionnaire and the examination-treatment information for analysis to the third-party organization are registered by a patient in the answer SV 3. In the example illustrated in FIG. 1, consent information indicating that the patient has confirmed the content of prior consent of the patient is contained in the answers of the patient.

The answer SV 3 receives answers of questionnaires daylily from the patient and accumulates answers. The answers to questionnaires contain personal information on the patient, such as living on treatment. The patient is able to confirm the content of answers of the patient in the past and confirm consent for provision to the third-party organization. In other words, the consent information presents, in addition to the examination-treatment information, consent for provision of the answers to questionnaires to the third-party organization.

Consent withdrawal information indicating withdrawal of consent for provision of the answers and the examination-treatment information to the third-party organization is sometimes registered in the answer SV 3 by the patient. For example, in a case where the initial purpose of the use in the third-party organization changes or the intention of the patient changes, or the like, the patient is able to register the consent withdrawal information. For example, in the example illustrated in FIG. 1, after confirming the answers of the patient in the past and the consent information, the patient is able to register consent withdrawal information in the answer SV 3 according to a procedure similar to that for registering answers and consent information.

The provision device 10 acquires patient information containing contact information on each patient from the patient information management SV 1 and stores the patient information in a storage unit. The provision device 10 acquires examination-treatment information on each patient from the examination-treatment information management SV 2 and stores the medical information in the storage unit. The provision device 10 acquires answers of a patient and consent information or consent withdrawal information from the answer SV 3 and registers the answers and the consent information or the consent withdrawal information in the patient information in the storage unit.

When the patient information contains consent information, the provision device 10 generates provision information by combining the patient information and the examination-treatment information on the patient and stores the provision information in the storage unit. When the patient information contains consent withdrawal information, the provision device 10 does not generate provision information or deletes provision information that is stored in the storage unit.

When data for analysis is requested by the third-party organization, the provision device 10 provides provision information in the storage unit. In this manner, the provision device 10 provides only electric data for which consent of the patient is obtained to the third-party organization, which makes it possible to safely provide electric data containing collected personal information for analysis.

### Configuration of Provision Apparatus

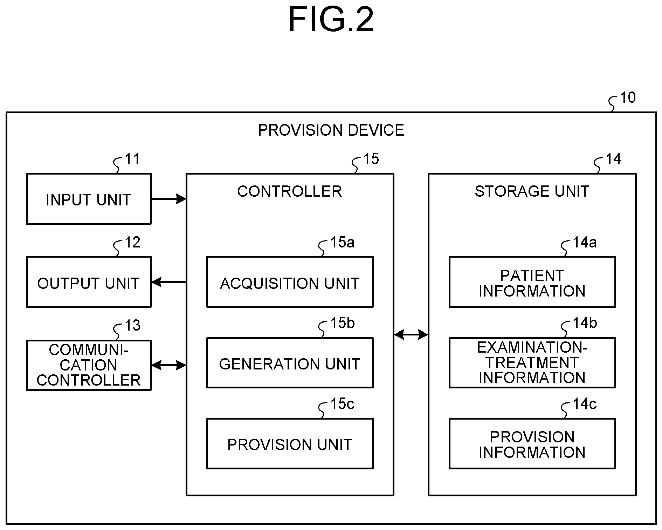

FIG. 2 is a schematic diagram exemplifying a schematic configuration of a provision device. As exemplified in FIG. 2, the provision device 10 is realized by a general-purpose computer, such as a personal computer, and includes an input unit 11, an output unit 12, a communication controller 13, a storage unit 14, and a controller 15.

The input unit 11 is realized by an input device, such as a keyboard and a mouse and inputs various types of instruction information for start of processing, or the like, to the controller 15 according to an input operation performed by an operator. The output unit 12 is realized by a display device, such as a liquid crystal display, a printing apparatus, such as a printer, or the like.

The communication controller 13 is realized by a network interface card (NIC), or the like, and controls communication between an external device, such as a server, and the controller 15 via a network. For example, the communication controller 13 controls communication between the patient information management SV 1, the examination-treatment information management SV 2, and the answer SV 3 and the controller 15.

The storage unit 14 is realized by a semiconductor memory device, such as a random access memory (R_AM) or a flash memory, or a storage device, such as a hard disk or an optical disk. In the embodiment, in the storage unit 14, for example, patient information 14a and examination-treatment information 14b that are used for a provision process to be described below, provision information 14c that is generated in the provision process, a result of the provision process, etc., are stored. The storage unit 14 may be configured to communicate with the controller 15 via the communication controller 13.

The patient information 14a is information on each patient. Specifically, as described above, the patient information 14a includes a patient number, first and last names, a birth date, gender, contact information, etc. An acquisition unit 15a to be described below acquires patient information from the patient information management SV 1 as initial data of consent data previously and stores the patient information in the storage unit 14. Answer data containing consent information that the acquisition unit 15a acquires from the answer SV 3 is added to the patient information 14a. As described above, the patient information 14a sometimes contain answers to a questionnaire containing personal information that are received daylily from the patient as described above. A medical institute No. that identifies a medical institute may be contained in the patient number.

The examination-treatment information 14b is information on examination and treatment on each patient. Specifically, as described above, the examination-treatment information 14b contains a patient number, first and last names, a birth date, gender, examination-treatment data, such as content of treatment and medication information, etc. Similarly, the acquisition unit 15a acquires examination-treatment information from the examination-treatment information from the examination-treatment information management SV 2 and stores the examination-treatment information in the storage unit 14 previously. Also in examination-treatment information, a medical institute No. that identifies a medical institute may be contained in the patient number.

As described above, the storage unit 14 stores the patient information 14a and the examination-treatment information 14b dispersedly. Specifically, the storage unit 14 stores each of the patient information 14a and the examination-treatment information 14b in a state of encrypted secret sharing. Accordingly, the provision device 10 safely manages the patient information 14a containing personal information, such as contact information and answers to a questionnaire, and the examination-treatment information 14b containing personal information, such as examination-treatment data, dispersedly.

The controller 15 is realized using a central processing unit (CPU), or the like, and executes a process program that is stored in a memory. Accordingly, as exemplified in FIG. 2, the controller 15 functions as the acquisition unit 15a, a generation unit 15b and a provision unit 15c. Each of or part of these functional units may be implemented in different hardware. For example, the provision unit 15c may be implemented in a device different from the acquisition unit 15a and the generation unit 15b. The controller 15 may include another functional unit.

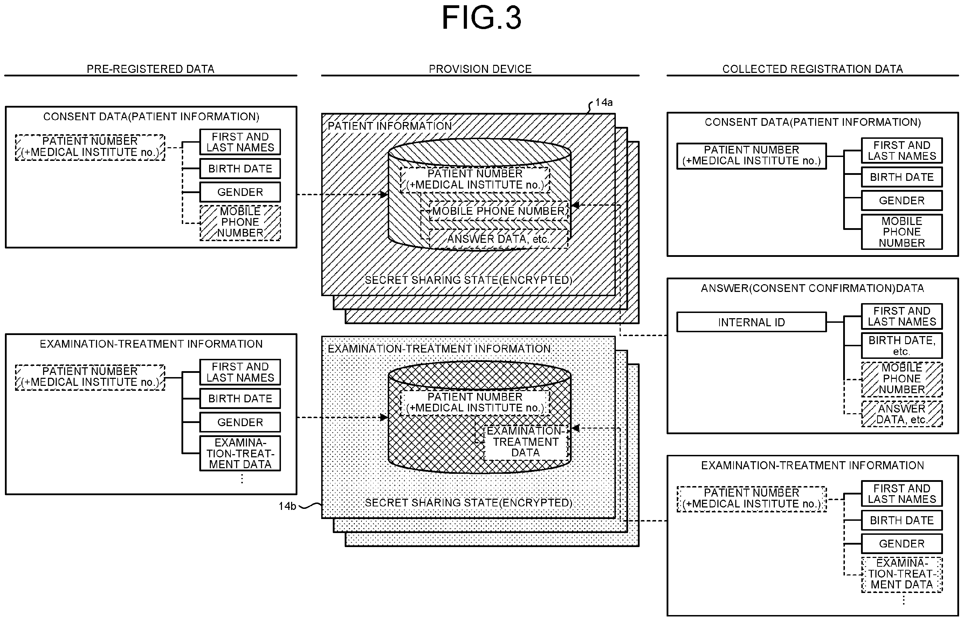

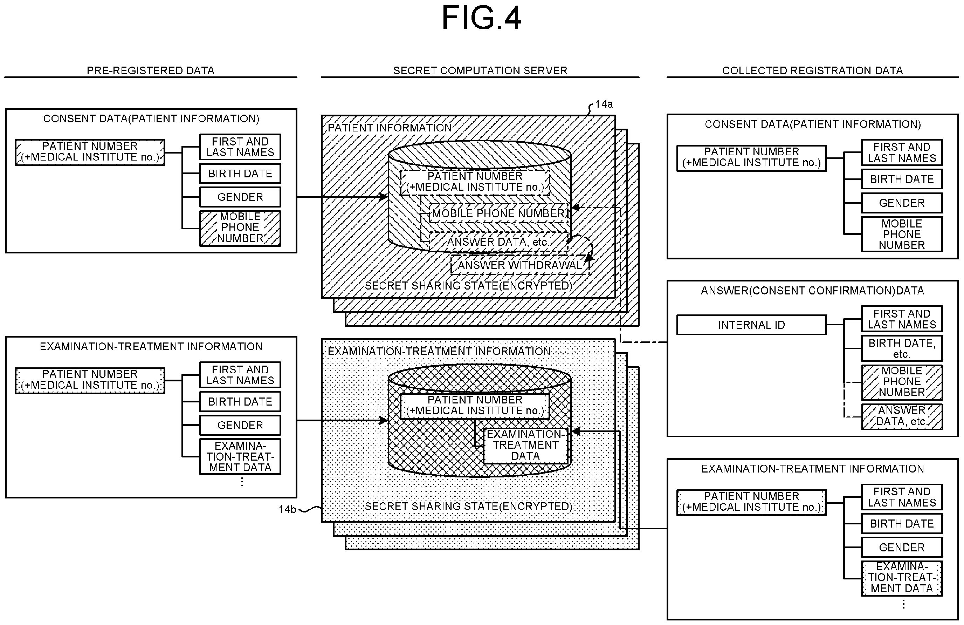

Next, FIG. 3 and FIG. 4 are diagrams for explaining a process by the acquisition unit. As illustrated in FIG. 3, the acquisition unit 15a previously acquires patient information from the patient information management SV 1 as initial data of consent data and stores the patient information in the storage unit 14. Similarly, the acquisition unit 15a previously acquires examination-treatment information from the examination-treatment information management SV 2 and stores the examination-treatment information in the storage unit 14.

The acquisition unit 15a acquires consent information indicating consent for provision of the patient information 14a and the examination-treatment information 14b to the third-party organization and adds the consent information to the patient information 14a in the storage unit 14. Specifically, the acquisition unit 15a acquires answers of a patient to a questionnaire for a follow-up at home and consent information indicating that previous consent for provision of answers to a questionnaire and examination-treatment information for analysis to the third-party organization has been confirmed from the answer SV 3. In the example illustrated in FIG. 3, consent information is contained in answer data.

The acquisition unit 15a adds the acquired answer data containing the consent information to the patient information 14a. The acquisition unit 15a specifies the patient information 14a using contact information, such as a mobile phone number, as key information and adds the acquired answer data.

The acquisition unit 15a further acquires consent withdrawal information indicating withdrawal of consent of each patient and adds the consent withdrawal information to the patient information 14a in the storage unit 14. Specifically, as illustrated in FIG. 4, the acquisition unit 15a acquires the consent withdrawal information instead of answer data from the answer SV 3. For example, the acquisition unit 15a acquires consent withdrawal information that that the patient registers in the answer SV 3 according to a procedure similar to that for registering answers and consent information after confirming the answers of the patient in the past and the consent information in a case where the initial purpose of the use in the third-party organization changes or the intention of the patient changes, or the like.

The acquisition unit 15a adds the acquired consent withdrawal information to the patient information 14a. The acquisition unit 15a specifies the patient information 14a using contact information, such as a mobile phone number, as key information and adds the acquired consent withdrawal information.

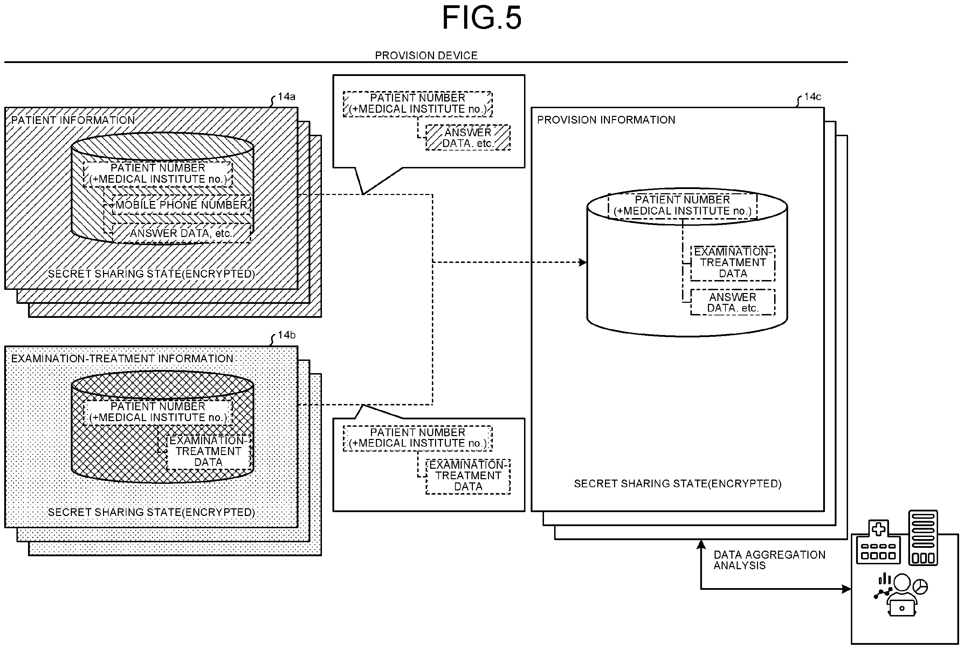

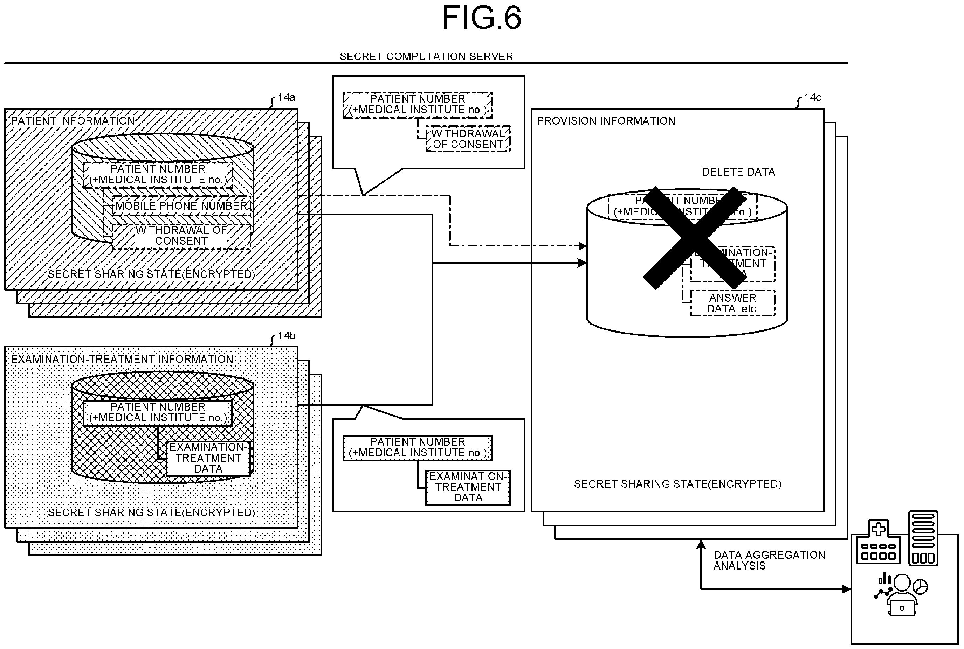

Next, FIG. 5 and FIG. 6 are diagrams for describing a process by the generation unit. The generation unit 15b generates the provision information 14c obtained by combining the patient information 14a containing consent information and the examination-treatment information 14b with respect to each patient. For example, as illustrated in FIG. 5, when answer data containing consent information is contained in the patient information 14a, the generation unit 15b specifies the examination-treatment information 14b corresponding to the patient information 14a using the patient number as key information and generates the provision information 14c.

As described above, the patient information 14a and the examination-treatment information 14b are stored safely in a state of encrypted secret sharing. Accordingly, the provision information 14c containing personal information is generated only when consent of the patient is obtained.

The generation unit 15b deletes contact information. such as a mobile phone number, from the generated provision information 14c. This makes it possible to prevent the contact information of the patient that is unnecessary for analysis from leaking to the third-party organization.

The generation unit 15b may store the generated provision information 14c in the storage unit 14. Alternatively, the generation unit 15b may output the generated provision information 14c to the provision unit 15c without storing the provision information 14c in the storage unit 14. In this case, for example, the generation unit 15b generates the provision information 14c when a request from the third-party organization is sensed and outputs the provision information 14c to the provision unit 15c.

The generation unit 15b stops the combining with the examination-treatment information 14b corresponding to the patient information containing consent withdrawal information. Specifically, as illustrated in FIG. 6, the generation unit 15b stores the generated provision information 14c in the storage unit 14 and deletes the provision information 14c corresponding to the patient information 14a containing the consent withdrawal information from the storage unit 14. In the example illustrated in FIG. 6, when consent withdrawal information is contained in the patient information 14a, the generation unit 15b specifies the provision information 14c corresponding to the patient information 14a and deletes the provision information 14c from the storage unit 14.

Go back to the description in FIG. 2. In response to a request from the third-party organization, the provision unit 15c provides the generated provision information 14c to the third-party organization. For example, the provision unit 15c provides the provision information 14c on the patient that meets requirements for being analyzed, such as the generation and the type of medication, among the provision information 14c with consent of each patient that is stored in the storage unit 14 to the third-party organization.

Accordingly, the provision device 10 is able to manage the patient information 14a and the examination-treatment information 14b dispersedly, generate the provision information 14c by combining the patient information 14a and the examination-treatment information 14b only when consent of the patient is obtained, and provide the provision information 14c to the third-party organization.

### Procedure of Provision Process

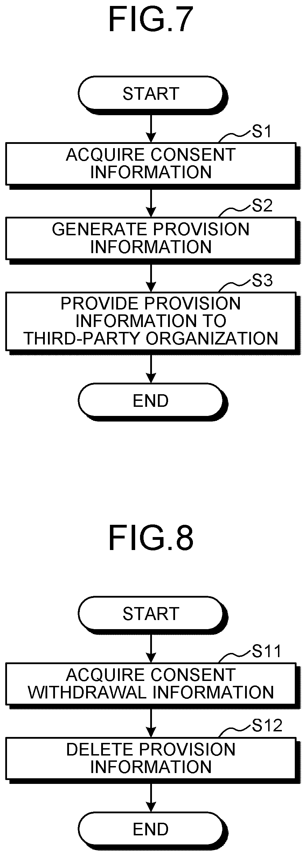

Next, with reference to FIG. 7 and FIG. 8, the provision process performed by the provision device 10 according to the embodiment will be described. FIG. 7 and FIG. 8 are flowcharts illustrating the procedure of the provision process. The provision process of the embodiment includes a generation process and a deletion process. First of all, FIG. 7 illustrates a procedure of the generation process. The flowchart in FIG. 7, for example, is started at the timing of an input of an instruction to start the generation process.

First of all, the acquisition unit 15a acquires consent information indicating consent for provision of the patient information 14a and the examination-treatment information 14b from each patient to the third-party organization and adds the consent information to the patient information 14a in the storage unit 14 (step S1). Specifically, the acquisition unit 15a acquires, from the answer SV 3, in addition to answers of the patient to a questionnaire for a follow-up at home, consent information indicating that the patient has confirmed prior consent of the patient for provision of the answers of the questionnaire and the examination-treatment information for analysis to the third-party organization.

Next, the generation unit 15b generates the provision information 14c obtained by combining the patient information 14a containing the consent information and the examination-treatment information 14b with respect to each patient (step S2). For example, when answer data containing consent information is contained in the patient information 14a, the generation unit 15b specifies the examination-treatment information 14b corresponding to the patient information 14a using a patient number as key information and generates the provision information 14c.

In response to a request from the third-party organization, the provision unit 15c provides the generated provision information 14c to the third-party organization (step S3). Accordingly, the generation process ends.

Next, FIG. 8 illustrates the procedure of the deletion process. The flowchart in FIG. 8, for example, is started at the timing of an input of an instruction to start a cancellation process.

First of all, the acquisition unit 15a acquires consent withdrawal information indicating withdrawal of consent of each patient and adds the consent withdrawal information to the patient information 14a in the storage unit 14 (step S11). For example, the acquisition unit 15a acquires, from the answer SV 3, consent withdrawal information that the patient registers in the answer SV 3 in a case where the initial purpose of the use in the third-party organization changes or the intention of the patient changes, or the like.

The generation unit 15b deletes the provision information 14c corresponding to the patient information 14a containing the consent withdrawal information from the storage unit 14 (step S12). For example, when consent withdrawal information is contained in the patient information 14a, the generation unit 15b specifies the provision information 14c corresponding to the patient information 14a using the patient number as key information and deletes the provision information 14c from the storage unit 14. Accordingly, the deletion process ends.

As described above, in the provision device 10 of the embodiment described above, the storage unit 14 stores the patient information 14a on each patient and the examination-treatment information 14b on examination and treatment on each patient dispersedly. The acquisition unit 15a acquires consent information indicating consent for provision of the patient information and the examination-treatment information from each patient to a third-party organization and adds the consent information to the patient information 14a in the storage unit 14. The generation unit 15b generates the provision information 14c obtained by combining the patient information 14a containing the consent information and the examination-treatment information 14b with respect to each patient. The provision unit 15c provides the generated provision information 14c to the third-party organization in response to a request from the third-party organization.

In this manner, the provision device 10 is able to manage the patient information and the examination-treatment information dispersedly, generate provision information by combining the patient information and the examination-treatment information only when consent of the patient is gained, and provide the provision information to the third-party organization for analysis. Accordingly, the provision device 10 is able to safely provide electric data containing collected personal information for analysis.

The storage unit 14 stores the patient information 14a and the examination-treatment information 14b in a state of encrypted secret sharing. This makes it possible to manage the electric data before obtainment of consent of the patient more safely. Accordingly, the provision information 14c containing personal information is generated by the provision device 10 more assuredly and safely only when consent of the patient is obtained.

The acquisition unit 15a further acquires consent withdrawal information indicating withdrawal of the consent of each patient and adds the consent withdrawal information to the patient information in the storage unit 14. In this case, the generation unit 15b stops the combining with the examination-treatment information 14b corresponding to the patient information 14a containing the consent withdrawal information. For example, the generation unit 15b stores the generated provision information 14c in the storage unit 14 and deletes the provision information 14c corresponding to the patient information 14a containing the consent withdrawal information from the storage unit 14. Accordingly, the provision device 10 is able to more assuredly generate the provision information 14c of only electric data on which consent of the patient is obtained for analysis.

### System Configuration, etc.

Each component of each device illustrated in the drawings is a functional idea and need not necessarily be configured physically as illustrated in the drawings. In other words, specific modes of distribution and integration of devices are not limited to those illustrated in the drawings and all or part of the devices can be configured by functional or physical distribution or integration in any unit according to various types of load and usage. Furthermore, all or given part of each processing function implemented by each device can be realized by a CPU or a graphics processing unit (GPU) and a program that is analyzed and executed by the CPU or the GPU or can be realized as hardware according to a wired logic.

Among the processes described in the above-described embodiment, all or part of the process that is described as one performed automatically can be performed manually or all or part of the process that is described as one performed manually can be performed automatically by a known method. In addition to this, the process procedure, the control procedure, the specific names, and the information including various types of data and parameters that are presented in the description above and the drawings are changeable freely unless otherwise noted.

### Program

It is possible to create a program in which the process that provision device described in the above-described embodiment executes is written in a computer-executable language. For example, it is also possible to create a program in which the process executed by the provision device 10 according to the embodiment is written in a computer-executable language. In this case, execution of the program by a computer makes it possible to obtain the same effect as that of the above-described embodiment. Furthermore, the program may be recorded in a computer-readable recording medium and a computer may be caused to read and execute the program that is recorded in the recording medium, thereby realizing the same process as that of the above-described embodiment.

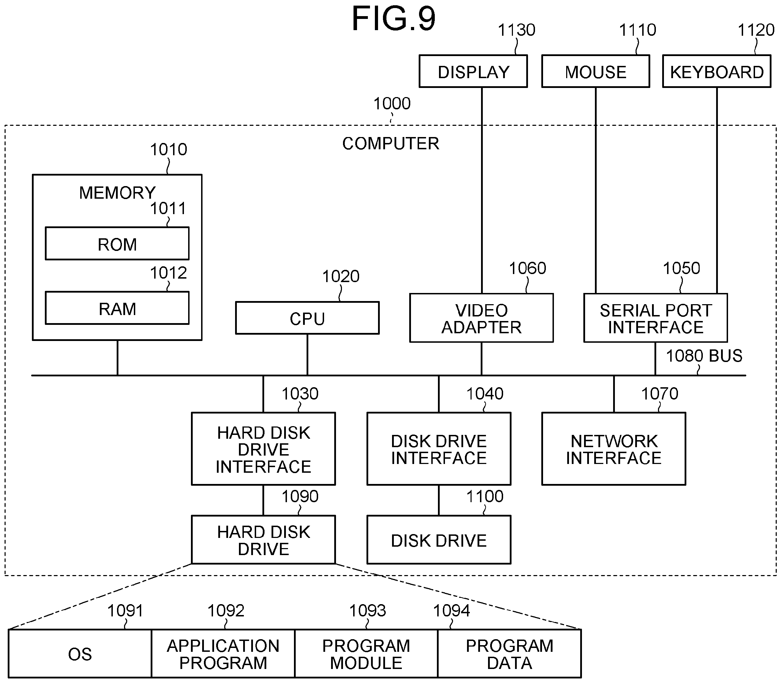

FIG. 9 is a diagram illustrating an example of the computer that executes a provision program. A computer 1000 includes, for example, a memory 1010, a CPU 1020, a hard disk drive interface 1030, a disk drive interface 1040, a serial port interface 1050, a video adapter 1060, and a network interface 1070. Each unit is connected via a bus 1080.

The memory 1010 includes a read only memory (ROM) 1011 and a RAM 1012. The ROM 1011 stores, for example, a boot program, such as a basic input output system (BIOS). The hard disk drive interface 1030 is connected to a hard disk drive 1090. The disk drive interface 1040 is connected to a disk drive 1100. For example, a detachable recording medium, such as a magnetic disk or an optical disk, is inserted into the disk drive 1100. For example, a mouse 1110 and a keyboard 1120 are connected to the serial port interface 1050. For example, a display 1130 is connected to the video adapter 1060.

The hard disk drive 1090 stores, for example, an OS 1091, an application program 1092, a program module 1093, and program data 1094. Each set of information described in the above-described embodiment is stored in, for example, the hard disk drive 1090 or the memory 1010.

The provision program, for example, is stored in the hard disk drive 1090 as the program module 1093 in which commands that are executed by the computer 1000 are written. Specifically, the program module 1093 in which each process executed by the provision device 10 described in the above-described embodiment is written is stored in the hard disk drive 1090.

The data that is used for information processing performed using the provision program is stored as the program data 1094 in, for example, the hard disk drive 1090. The CPU 1020 reads the program module 1093 and the program data 1094 that are stored in the hard disk drive 1090 into the RAM 1012 as requested and executes each of the procedures described above.

The program module 1093 and the program data 1094 according to the program are not limited to being stored in the hard disk drive 1090, and the program module 1093 and the program data 1094 may be stored in, for example, a detachable storage medium and may be read by the CPU 1020 via the disk drive 1100, or the like. Alternatively, the program module 1093 and the program data 1094 according to the provision program may be stored in another computer that is connected via a network, such as a local area network (LAN) or a wide area network (WAN), and may be read by the CPU 1020 via the network interface 1070.

The embodiment to which the invention made by the inventors has been described; however, the invention is not limited by the description and the drawings that form part of the disclosure of the invention according to the embodiment. In other words, other embodiments, examples, techniques to be practiced, etc., that are performed by those skilled in the art based on the embodiment are within the scope of the invention.

### [Explanation of Reference]

- 1: PATIENT INFORMATION MANAGEMENT SV
- 2: EXAMINATION-TREATMENT INFORMATION MANAGEMENT SV
- 3: ANSWER SV
- 10: PROVISION APPARATUS
- 11: INPUT UNIT
- 12: OUTPUT UNIT
- 13: COMMUNICATION CONTROLLER
- 14: STORAGE UNIT
- 14a: PATIENT INFORMATION
- 14b: EXAMINATION-TREATMENT INFORMATION
- 14c: PROVISION INFORMATION
- 15: CONTROLLER
- 15a: ACQUISITION UNIT
- 15b: GENERATION UNIT
- 15c: PROVISION UNIT

## Claims

1. A provision device comprising:
a storage unit configured to store patient information on each patient and examination-treatment information on examination and treatment on each patient dispersedly;
an acquisition unit configured to acquire consent information indicating consent for provision of the patient information and the examination-treatment information from each patient to a third-party organization and add the consent information to the patient information in the storage unit;
a generation unit configured to generate provision information obtained by combining the patient information containing the consent information and the examination-treatment information with respect to each patient; and
a provision unit configured to provide the generated provision information to the third-party organization in response to a request from the third-party organization.

2. The provision device according to claim 1, wherein the storage unit stores the patient information and the examination-treatment information in a state of encrypted secret sharing.

3. The provision device according to claim 1, wherein the acquisition unit acquires consent withdrawal information indicating withdrawal of the consent of each patient and adds the consent withdrawal information to the patient information in the storage unit, and
the generation unit stops combining with the examination-treatment information corresponding to the patient information containing the consent withdrawal information.

4. The provision device according to claim 3, wherein the generation unit stores the generated provision information in the storage unit and deletes the provision information corresponding to the patient information containing the consent withdrawal information from the storage unit.

5. A provision method executed by a provision device, the provision method comprising:
an acquiring step of referring to a storage unit that stores patient information on each patient and examination-treatment information on examination and treatment on each patient dispersedly, acquiring consent information indicating consent for provision of the patient information and the examination-treatment information from each patient to a third-party organization, and adding the consent information to the patient information in the storage unit;
a generating step of generating provision information obtained by combining the patient information containing the consent information and the examination-treatment information with respect to each patient; and
a providing step of providing the generated provision information to the third-party organization in response to a request from the third-party organization.

6. A provision program that causes a computer to execute:
an acquiring step of referring to a storage unit that stores patient information on each patient and examination-treatment information on examination and treatment on each patient dispersedly, acquiring consent information indicating consent for provision of the patient information and the examination-treatment information from each patient to a third-party organization, and adding the consent information to the patient information in the storage unit;
a generating step of generating provision information obtained by combining the patient information containing the consent information and the examination-treatment information with respect to each patient; and
a providing step of providing the generated provision information to the third-party organization in response to a request from the third-party organization.
